# EUROPEAN PATENT SPECIFICATION

(11) **EP 2 019 652 B1**
(45) Date of publication and mention of the grant of the patent: **07.07.2010**
(21) Application number: 07794743.0
(22) Date of filing: 10.05.2007
(51) Int. Cl.: A61F 2/24

(54) **DEVICE FOR REGULATING BLOOD FLOW**
VORRICHTUNG ZUR BLUTFLUSSREGULIERUNG
APPAREIL RÉGULATEUR DE DÉBIT SANGUIN

(30) Priority: 25.05.2006 US 808406 P; 31.05.2006 US 809483 P
(43) Date of publication of application: 04.02.2009
(73) Proprietor: Deep Vein Medical, Inc., Cambell Hall, NY 10916 (US)
(72) Inventor: KALMANN, Menno, NL-8075 Pd Elspeet (NL)
(74) Representative: Jackson, Derek Charles
(86) International application number: PCT/US2007/011318
(87) International publication number: WO 2007/139677

(56) References cited:
- WO-A-01/54625
- US-A- 5 500 014
- US-A1- 2002 099 439
- US-A1- 2004 093 070
- US-B1- 6 605 112

## Description

### BACKGROUND OF THE INVENTION

### 1. Field of the Invention

The subject invention is directed to a device for regulating blood flow in the venous system, and more particularly, to an implantable valve device for regulating the flow of blood through a blood vessel.

### 2. Description of Related Art

The blood system, and in particular the venous blood system of the legs and arms is provided with valves that are uniquely located in a manner so as to ensure that blood will not flow back upstream in the direction from which it has been pumped from the heart. In the arms and legs, there is a deep venous system and a surface venous system. Due to various causes, thrombosis can occur in the deep venous system. Blood thinning can alleviate this problem. However, valves do not effectively close and often leak when the blood in thinned. This can cause increased venous blood pressure in the direction of the ankles, which can lead to a variety of problems including varicose veins and open leg. Complaints of this type are wide spread among those who spend prolonged periods of time in a standing position, for instance, surgeons.

The surface venous system of the leg is relatively weaker than the deep venous system, and it has the tendency to spontaneously widen. This widening prevents the valves from functioning effectively and can lead to varicose veins, which are both unattractive and painful. Major surgery is often required to treat these blood vessel problems. For example, varicose veins are treated by either closing off the vein, which leads to a reduced blood flow capacity and increased pressure on surrounding blood vessels to ensure blood supply, or by completely removing the varicose veins, which leads to the same problem.

US-A-20040093070 describes a stent-based valve which includes a radially expandable structural frame comprising a proximal anchor and a distal anchor. The proximal and distal anchors are formed from a lattice of interconnected elements, and have a substantially cylindrical configuration with first and second open ends and a longitudinal axis extending therebetween. The stent based valve also comprises one or more connecting members, each having a first and a second end. The first end of each connecting member is attached to the proximal anchor and the second end of each connecting member is attached to the distal anchor. A biocompatible valve assembly is attached to the proximal anchor and extends distally along the one or more connecting members.

WO-A-0154625 describes a valve within a frame which comprises a self-expanding stent frame. The valve has at least one expandable and contractible pocket member within the stent frame for resisting and permitting fluid flow, respectively.

US-A-5 500014 describes a biological valvular prosthesis defined by a chemically fixed biological derived conduit having at least one integrally formed tissue valve. The chemical fixation of the valve ensures that the leaflets remain open under normal forward blood flow conditions, but close under minimal backflow pressure. The biological valvular prosthesis may also be in the form of a tubular conduit bearing one tissue valve.

US-A-20020099439 describes a device and method for replacing or restoring competence to incompetent valves. The device is constructed of a material capable of promoting cellular ingrowth and is preferably absorbable over time. The device is sized and arranged to mimic the valve it is replacing or repairing.

The subject invention is directed to a device for obviating problems of the type described above.

### SUMMARY OF THE INVENTION

According to the present invention there is provided an implantable device for regulating blood flow through a blood vessel, comprising:
a) an elongated frame dimensioned and configured to be implanted in a blood vessel, the frame including axially spaced apart ring portions, and at least one linking member connecting the axially spaced apart ring portions to one another; and
b) a valve membrane supported between the axially spaced apart ring portions of the frame and adapted for movement between a first position in which blood flow through the frame is permitted and a second position in which blood flow through the frame is inhibited,
wherein the valve membrane has a body portion and a narrow elongated neck portion which, in the first position, extends in a diametral direction of the device and, in the second position, extends in an axial direction of the device adjacent to a circumferential region thereof.

The linking member may extend from opposing sides of the ring portions. The linking member may be curved. Preferably, the frame includes a plurality of peripherally spaced apart linking members. The frame may be formed at least in part from a shape memory alloy material. The valve membrane is preferably formed at least in part from PTFE. The membrane may be coated at least in part with an anti-clotting agent. The neck portion of the membrane may be attached to one ring portion and the body portion may be attached to the other ring portion. The neck portion may have a length at least equal to the radius of the ring portions, for example greater than the radius of the ring portions, and preferably less than the diameter of the ring portions. The body portion and the neck portion of the membrane may be attached to their respective ring portions substantially 180 degrees apart.

These and other features of the subject invention will become more readily apparent to those having ordinary skill in the art from the following enabling description of the invention taken in conjunction with the drawings appended hereto.

### BRIEF DESCRIPTION OF THE DRAWINGS

So that those skilled in the art to which the subject invention appertains will readily understand how to make and use the apparatus of subject invention without undue experimentation, preferred embodiments thereof will be described in detail hereinbelow with reference to certain figures, wherein:
Fig. 1 is a perspective view of the frame of a flow-regulating device constructed in accordance with a preferred embodiment of the subject invention;
Fig. 2 is a plan view of a membrane employed with the frame of Fig. 1;
Fig. 3 is a schematic perspective view of the flow-regulating device of the subject invention, wherein the frame is simplified to illustrate the position of the membrane to permit blood flow through a vessel;
Fig. 4 is a schematic perspective view of the flow-regulating device of the subject invention as in Fig. 3, with the membrane oriented to block the flow of blood through a vessel;
Fig. 5 is a perspective view of a flow-regulating device not covered by the claims with a membrane oriented to block blood flow through a vessel;
Fig. 6 is a perspective view as in Fig. 5, with the membrane oriented to permit blood flow through a vessel;
Fig. 7 is a perspective view of another flow-regulating device not covered by the claims with a frame in a closed condition;
Fig. 8 is a perspective view of the device shown in Fig. 7, with the frame expanded into an open position;
Fig. 9 is a perspective view of the device of Fig. 7, shown with a balloon catheter prior to deployment in a blood vessel; and
Fig. 10 is a perspective view of the device of Fig. 7, shown with a balloon catheter inflated to expand the device into a deployed condition within a blood vessel.

### ENABLING DESCRIPTION OF PREFERRED EMBODIMENTS

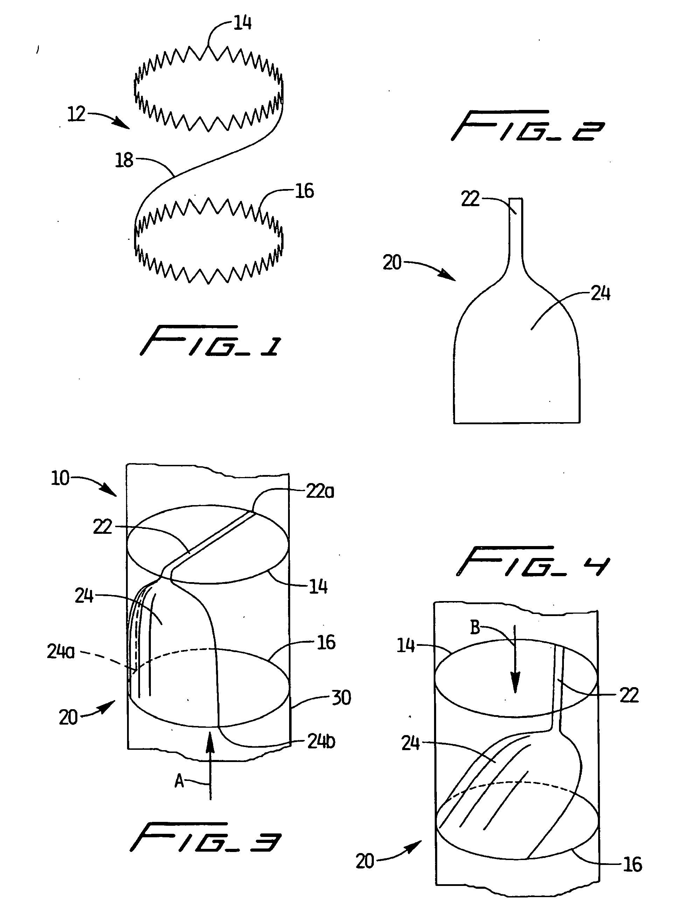

Referring now to the drawings wherein like reference numerals, there is illustrated in Fig 1, a flow regulating device constructed in accordance with a preferred embodiment of the subject invention, and designated generally by reference numeral 10. Regulating device 10 includes an elongated frame 12 that consists of upper and lower crown-shaped or wave-like rings 14 and 16. That is, each ring has connecting V-shapes as shown. These rings 14, 16 are preferably larger in diameter than the host vein in their expanded placement configuration, to ensure that the device remains in a desired position and orientation after implantation. For example, the diameter of the rings may be 1.25% larger than the diameter of the intended host vein.

Rings 14 and 16 are connected to one another by at least one connective or linking member in the form of a bar or wire 18. For ease of illustration, only one connective wire 18 is shown in Fig. 1. Bar or wire 18 is curved and adapted and configured to follow the circumference of the host vessel. Preferably, the bar or wire 18 is attached to the opposed rings 14 and 16 of frame 12 at locations that are about 180° apart from one another, as shown. This gives frame 12 an inherent flexibility and enables it to move with the natural movements (e.g., peristaltic) of the vein.

Due to the crown-shape of the rings 14 and 16, the device 10 can be reduced to approximately 1/5 of the final implanted diameter and could be introduced into a blood vessel through a relatively small delivery device. For example, a device having a working diameter of 2 mm to 2.667 mm (6F to 8F, French Catheter Scale) could be used.

As shown in Figs 3 and 4, wherein the connective rod(s) 18 are not shown and the rings 14, 16 are simplified for ease of illustration, device 10 includes a valve member 20 that is operatively associated with frame 12 for regulating the flow of blood through a vessel by moving between open and closed positions. Valve member 20 is formed from a sheet of ultra thin membrane material such as a non-expandable PTFE material or the like. It is envisioned that the membrane could be bonded or otherwise coated with an anti-clotting or anti-coagulant agent such as Heparin.

As best seen in Fig. 2, valve membrane 20 has a narrow elongated neck portion 22 for attachment to the upper ring 14 of frame 12 and a wide body portion 24 for attachment to the lower ring 16 of frame 12. The narrow neck portion 22 as shown extends across the diameter of the device, and preferably has a length greater than a radius of the device (ring) and slightly less than the diameter of the ring. The attachment locations (22a of neck portion 22 and 24a, 24b of body portion 24) of the membrane 20 on each ring are preferably approximately 180° degrees from one another so that the body portion 24 of the membrane 20 will extend substantially if not entirely across the expanse of frame in the closed position shown in Fig 4. Note the membrane could be attached to ring 16 along its curved perimeter or attached at specific points, e.g. 24a, 24b, or at additional points.

Referring to Fig. 3, blood flowing through the blood vessel 30 in the downstream direction indicted by arrow "A" will act against the valve membrane 20 in such a manner so as to push the wide body portion 14 ot the membrane 20 against the wall of the blood vessel 30. At such a time, blood will flow freely through the frame 12, impeded only incidentally by the narrow neck portion 22 of membrane 20 extending across the device.

Referring to Fig 4, blood flowing through the blood vessel 30 in the upstream direction indicated by arrow "B" will act against the valve membrane 20 in such a manner so as to push the wide body portion 24 in a direction as shown, substantially if not entirely closing off blood flow through the blood vessel 30. Due to the length of the narrow part 22 of the valve membrane 20, the wide body portion 24 will close at a relatively steep angle (e.g., 30°). This is important because the steeper the closure angle, the less force required to push the valve membrane back to an open position with the natural blood pressure.

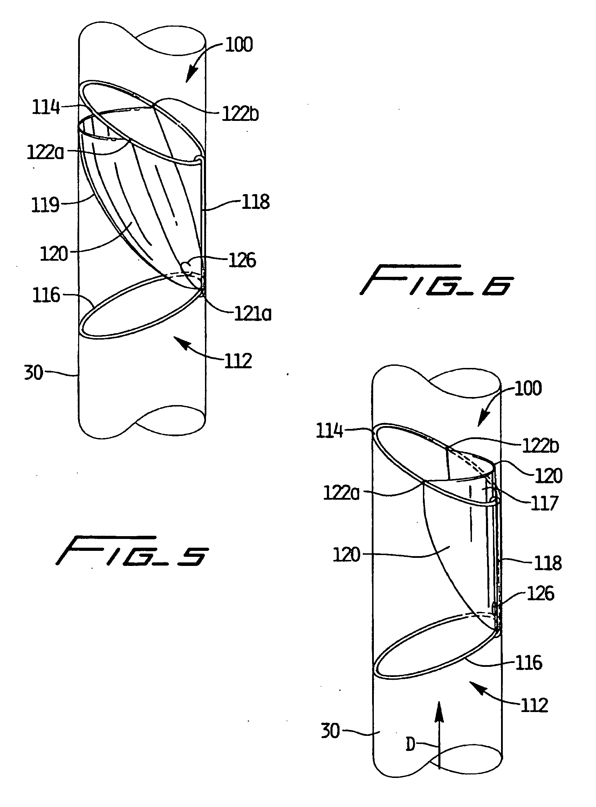

Referring now to Fig. 5, to minimize the number and complexity of implantable components, an implantable device 100 is provided that includes a frame 112 having two axially spaced apart substantially circular rings 114, 116 and a connecting bar 118, with an ultra thin, generally triangular shaped membrane 120 operatively associated therewith. As shown, each of the rings 114, 116 is positioned at an angle, preferably obtuse as shown, to the connecting bar 118. The lower apex of the triangular membrane 120 is attached to the lower ring portion 116 of frame 112 (attachment region 121a), and the upper apices of the triangular membrane 120 are attached to the upper ring portion 114 of frame 112 at diametrically opposed positions (attachment regions 122a, 122b). Preferably, the upper portion of the membrane 120 is loosely attached to the upper ring portion 114, allowing it to slide down the ring during insertion. The lower portion of the membrane 120 is attached to the lower ring portion 116 in the same general area as the connecting rod 118. The membrane has a curved or convex outer surface 119 in the flow blocking position of Figure 5 and a curved or convex outer surface 117 facing in the opposite direction (radially) in the blood flow position of Figure 6 (see arrow D).

It is envisioned that the frame 112 (and frame 12) is made from a shape-memory or super-elastic material such as Nitinol or a similar material, so as to enable the collapse and recovery of the rings during implantation in blood vessel 30. The ultra thin membrane 120 is preferably made from a material such as PTFE, and may be provided with an anti-clotting drug.

It is also envisioned that valve membrane 120 can have a small slit or hole 126 adjacent the lower apex of the membrane near the connection with lower ring portion 116 to allow some of the blood trapped behind to flow back through the membrane. This will reduce the likelihood of clotting.

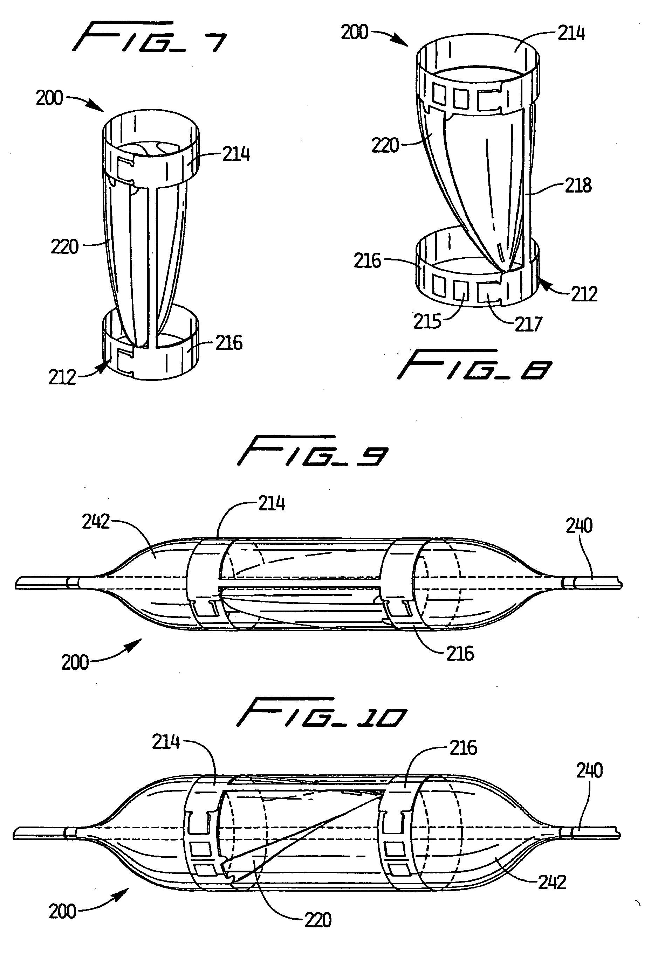

Referring now to Figs. 7 through 10, there is illustrated another embodiment of an implantable device, which is designated generally by reference numeral 200. Device 200 includes a frame 212 having opposed flexible straps 214, 216 and a connecting structure 218. Straps 214 and 216 are preferably formed from a shape memory material that is normally biased into a coiled or closed configuration, shown for example in Fig. 7. A generally triangular membrane 220 is attached to frame 212 in a manner similar to the way in which membrane 120 is attached to frame 112. The rings 214, 216 of frame 212 are adapted and configured for securement in an expanded or open position, shown in Fig. 8, through the interaction of a locking tangs 217 and apertures 215.

As illustrated in Figs. 9 and 10, device 200 is implanted in a blood vessel using a balloon catheter 240. More particularly, rings 214 and 216 are moved from a closed position to an expanded position by inflating balloon 242. Upon expansion, to a desired position, tangs 217 engage apertures 215 to lock the rings 214 and 216 in a desired position. The balloon 242 is then deflated and the catheter 240 is removed from the blood vessel so the device 200 can regulate the flow of blood through the vessel, in the manner described previously with respect to device 100.

Although the blood flow-regulating device of the subject invention has been described with respect to a preferred embodiment, those skilled in the art will readily appreciate that changes and modifications may be made thereto without departing from the scope of the claims.

## Claims

1. An implantable device for regulating blood flow through a blood vessel, comprising:
a) an elongated frame (12) dimensioned and configured to be implanted in a blood vessel, the frame including axially spaced apart ring portions (14, 16), and at least one linking member (18) connecting the axially spaced apart ring portions to one another; and
b) a valve membrane (20) supported between the axially spaced apart ring portions of the frame and adapted for movement between a first position in which blood flow through the frame is permitted and a second position in which blood flow through the frame is inhibited,
**characterised in that** the valve membrane has a body portion (24) and a narrow elongated neck portion (22), the neck portion, in the first position, extends in a diametral direction of the device and, in the second position, extends in an axial direction of the device adjacent to a circumferential region thereof.

2. A device as claimed in claim 1, wherein the linking member (18) extends from opposing sides of the ring portions (14, 16).

3. A device as claimed in claim 1 or 2, wherein the linking member (18) is curved.

4. A device as claimed in any preceding claim, wherein the frame (12) includes a plurality of peripherally spaced apart linking members (18).

5. A device as claimed in any preceding claim, wherein the frame (12) is formed at least in part from a shape memory alloy material.

6. A device as claimed in any preceding claim, wherein the valve membrane (20) is formed at least in part from PTFE.

7. A device as claimed in any preceding claim, wherein the valve membrane (20) is coated at least in part with an anti-clotting agent.

8. A device as claimed in any preceding claim, wherein the neck portion (22) of the membrane (20) is attached to one ring portion (14) and the body portion (24) is attached to the other ring portion (16).

9. A device as claimed in any preceding claim , wherein the neck portion (22) has a length at least equal to the radius of the ring portions (14, 16).

10. A device as claimed in claim 9, wherein the neck portion (22) has a length greater than the radius of the ring portions (14, 16).

11. A device as claimed in claim 9 or 10, wherein the neck portion (22) has a length less than the diameter of the ring portions (14, 16).

12. A device as claimed in any preceding claim, wherein the body portion (24) and the neck portion (22) of the membrane (20) are attached to their respective ring portions (14, 16) substantially 180 degrees apart.

## Patentansprüche

1. Implantierbare Vorrichtung zur Regulierung des Blutflusses durch ein Blutgefäß, wobei die Vorrichtung Folgendes umfasst:
a) ein gestrecktes Gestell (12), das zum Implantieren in einem Blutgefäß bemessen und konfiguriert ist, wobei das Gestell axial beabstandete Ringabschnitte (14, 16) und mindestens ein Verbindungselement (18) beinhaltet, das die axial beabstandeten Ringabschnitte miteinander verbindet; und
b) eine Ventilmembran (20), die zwischen den axial beabstandeten Ringabschnitten des Gestells getragen wird und zur Bewegung zwischen einer ersten Position, in der Blutfluss durch das Gestell zugelassen wird, und einer zweiten Position, in der Blutfluss durch das Gestell gehemmt wird, eingerichtet ist,
**dadurch gekennzeichnet, dass** die Ventilmembran einen Körperabschnitt (24) und einen schmalen gestreckten Halsabschnitt (22) aufweist, wobei der Halsabschnitt sich in der ersten Position in einer diametralen Richtung der Vorrichtung erstreckt und sich in der zweiten Position in einer axialen Richtung der Vorrichtung angrenzend an einen Umfangsbereich davon erstreckt.

2. Vorrichtung nach Anspruch 1, wobei das Verbindungselement (18) sich von gegenüberliegenden Seiten der Ringabschnitte (14, 16) erstreckt.

3. Vorrichtung nach Anspruch 1 oder 2, wobei das Verbindungselement (18) gebogen ist.

4. Vorrichtung nach einem der vorhergehenden Ansprüche, wobei das Gestell (12) mehrere peripher beabstandete Verbindungselemente (18) beinhaltet.

5. Vorrichtung nach einem der vorhergehenden Ansprüche, wobei das Gestell (12) zumindest zum Teil aus einem Formgedächtnislegierungsmaterial ausgebildet ist.

6. Vorrichtung nach einem der vorhergehenden Ansprüche, wobei die Ventilmembran (20) zumindest zum Teil aus PTFE ausgebildet ist.

7. Vorrichtung nach einem der vorhergehenden Ansprüche, wobei die Ventilmembran (20) zumindest zum Teil mit einer gerinnungshemmenden Substanz beschichtet ist.

8. Vorrichtung nach einem der vorhergehenden Ansprüche, wobei der Halsabschnitt (22) der Membran (20) an einem Ringabschnitt (14) befestigt ist und der Körperabschnitt (24) an dem anderen Ringabschnitt (16) befestigt ist.

9. Vorrichtung nach einem der vorhergehenden Ansprüche, wobei der Halsabschnitt (22) eine Länge aufweist, die zumindest gleich dem Radius der Ringabschnitte (14, 16) ist.

10. Vorrichtung nach Anspruch 9, wobei der Halsabschnitt (22) eine Länge aufweist, die größer als der Radius der Ringabschnitte (14, 16) ist.

11. Vorrichtung nach Anspruch 9 oder 10, wobei der Halsabschnitt (22) eine Länge aufweist, die kleiner als der Durchmesser der Ringabschnitte (14, 16) ist.

12. Vorrichtung nach einem der vorhergehenden Ansprüche, wobei der Körperabschnitt (24) und der Halsabschnitt (22) der Membran (20) im Wesentlichen um 180 Grad voneinander beabstandet an ihren jeweiligen Ringabschnitten (14, 16) befestigt sind.

## Revendications

1. Dispositif implantable pour réguler le flux sanguin à travers un vaisseau sanguin, comprenant :
a) un cadre allongé (12) dimensionné et configuré pour être implanté dans un vaisseau sanguin, le cadre incluant des portions annulaires (14, 16) espacées axialement et au moins un élément de liaison (18) connectant les portions annulaires espacées axialement l'une à l'autre ; et
b) une membrane à clapet (20) supportée entre les portions annulaires espacées axialement du cadre et adaptée au mouvement entre une première position dans laquelle le flux sanguin à travers le cadre est permis, et une seconde position dans laquelle le flux sanguin à travers le cadre est interdit,
**caractérisé en ce que** la membrane à clapet possède une portion de corps (24) et une portion de cou étroite allongée (22), la portion de cou s'étend, dans la première position, dans une direction diamétrale du dispositif et s'étend, dans la seconde position, dans une direction axiale du dispositif de manière adjacente à une région périphérique de celui-ci.

2. Dispositif selon la revendication 1, dans lequel l'élément de liaison (18) s'étend depuis des côtés opposés des portions annulaires (14, 16).

3. Dispositif selon la revendication 1 ou 2, dans lequel l'élément de liaison (18) est courbé.

4. Dispositif selon l'une quelconque des revendications précédentes, dans lequel le cadre (12) inclut une pluralité d'éléments de liaison (18) espacés de manière périphérique.

5. Dispositif selon l'une quelconque des revendications précédentes, dans lequel le cadre (12) est formé au moins en partie d'un matériau en alliage à mémoire de forme.

6. Dispositif selon l'une quelconque des revendications précédentes, dans lequel la membrane à clapet (20) est formée au moins en partie de PTFE.

7. Dispositif selon l'une quelconque des revendications précédentes, dans lequel la membrane à clapet (20) est enduite au moins en partie d'un agent anti-coagulant.

8. Dispositif selon l'une quelconque des revendications précédentes, dans lequel la portion de cou (22) de la membrane (20) est reliée à une portion annulaire (14) et la portion de corps (24) est reliée à l'autre portion annulaire (16).

9. Dispositif selon l'une quelconque des revendications précédentes, dans lequel la portion de cou (22) possède une longueur au moins égale au rayon des portions annulaires (14, 16).

10. Dispositif selon la revendication 9, dans lequel la portion de cou (22) possède une longueur supérieure au rayon des portions annulaires (14, 16).

11. Dispositif selon la revendication 9 ou 10, dans lequel la portion de cou (22) possède une longueur inférieure au diamètre des portions annulaires (14, 16).

12. Dispositif selon l'une quelconque des revendications précédentes, dans lequel la portion de corps (24) et la portion de cou (22) de la membrane (20) sont reliées à leurs portions annulaires respectives (14, 16) essentiellement à 180 degrés l'une de l'autre.
